# EUROPEAN PATENT APPLICATION

(11) **EP 4 652 867 A1**
(43) Date of publication of application: **26.11.2025**
(21) Application number: 24177550.1
(22) Date of filing: 23.05.2024
(51) Int. Cl.: A24F 40/05, A24F 40/85, A24F 40/10, A24F 40/50, A61M 11/00

(54) **AEROSOL GENERATING APPARATUS**

(71) Applicant: Imperial Tobacco Limited, Bristol, BS3 2LL (GB)
(72) Inventor: The designation of the inventor has not yet been filed
(74) Representative: Mewburn Ellis LLP

(57) **Abstract**

There is provided a method of controlling an aerosol generating apparatus (1) to modify the delivery of aerosol generated by the aerosol generating apparatus, wherein the aerosol generating apparatus includes: a tank (32) for containing an aerosol precursor (6), a piezoelectric transducer (100) arranged to receive aerosol precursor from the tank and configured to induce cavitation in the received aerosol precursor to thereby generate an aerosol from the received aerosol precursor. The method comprises: providing, to the piezoelectric transducer (100), a surface priming signal to drive the piezoelectric transducer so as to expel excess aerosol precursor from a surface of the piezoelectric transducer.

## Description

### FIELD

The present disclosure relates to an aerosol generating apparatus.

### BACKGROUND

A typical aerosol generating apparatus may comprise a power supply, an aerosol generating unit that is driven by the power supply, an aerosol precursor, which in use is aerosolised by the aerosol generating unit to generate an aerosol, and a delivery system for delivery of the aerosol to a user.

In some cases, the aerosol generating unit may include an ultrasonic generator e.g. a piezoelectric transducer (PET) for generating the aerosol. In use, the surface of the PET will expand and contract as it vibrates.

A PET generates aerosol by causing cavitation to occur within a liquid aerosol precursor that is provided on a surface of the PET. Cavitation refers to the phenomenon where the static pressure of a liquid reduces to below the liquid's vapour pressure, leading to the formation of small vapour filled cavities within the liquid. When the cavities are subsequently subjected to a higher pressure, the cavities collapse resulting in a shock wave that propagates through the liquid. This shock wave induces capillary waves, or ripples, in a surface distal (referred to herein as the upper surface of the liquid) from the PET that may form ligaments to expel droplets from the upper surface. More succinctly, in a thin layer of liquid, the collapsing of the cavities can induce a disturbance in the liquid that causes liquid droplets to be expelled from liquid, thereby forming an aerosol over the surface of the liquid, typically within an aerosolization chamber.

In the context of a PET for generating the aerosol, when the surface of the PET expands, the liquid on the surface will conform to the expanded surface. When the surface of the PET subsequently contracts, the static pressure in the liquid will fall as it is effectively dragged with the surface with the decrease in static pressure being proportional to the speed of the movement of the PET surface (i.e., the frequency of the vibration). If the frequency and amplitude of vibration of the PET is sufficiently high, cavitation will occur as a result of the contraction.

When the surface of the PET subsequently expands, the static pressure in the liquid will rise as it is effectively compressed by the surface with the increase in static pressure being proportional to the speed of the movement of the PET surface (i.e., the frequency of the vibration). Any cavities in the liquid previously formed may then implode, generating shock waves in the liquid capable of expelling droplets to form an aerosol.

In an aerosol generating apparatus using a PET, a liquid aerosol precursor is typically applied to the PET surface using a wick in fluid communication with a tank. The aerosol generated by cavitation of the liquid aerosol precursor will be drawn from the aerosolization chamber along an aerosol flow path by suction at a mouthpiece outlet.

Aerosol generating apparatuses that use a PET for generating the aerosol present numerous challenges, including accurately driving the vibrational element and inefficiencies in the requisite circuitry.

In spite of the effort already invested in the development of aerosol generating apparatuses/systems further improvements are desirable.

### SUMMARY

In an aspect, the present disclosure provides a method of controlling an aerosol generating apparatus to modify the delivery of aerosol generated by the aerosol generating apparatus. The aerosol generating apparatus includes: a tank for containing an aerosol precursor, and a piezoelectric transducer arranged to receive aerosol precursor from the tank. The piezoelectric transducer is configured to induce cavitation in the received aerosol precursor to thereby generate an aerosol from the received aerosol precursor.

The method comprises: providing, to the piezoelectric transducer, a surface priming signal to drive the piezoelectric transducer so as to expel excess aerosol precursor from a surface of the piezoelectric transducer.

The surface of the piezoelectric transducer may be considered to be a surface of the piezoelectric transducer that receives aerosol precursor from the tank. Upon application of a current through the piezoelectric transducer, the transducer is configured to vibrate, causing displacement/distortion of the surface upon which the aerosol precursor is received. The displacement/distortion of the surface of the piezoelectric transducer induces cavitation in the and a consequent shock in the received aerosol precursor, thereby generating an aerosol. The mechanism for such aerosol generation has been described, for example, in Kooij et al. Sci. Rp., 9, 6128 (2019), the entirety of which is incorporated herein by reference.

One or more parameters of the aerosol precursor may impact one or more parameters of the aerosol generated by the aerosol generating apparatuses described herein.

The density, viscosity, volume and molecular size of aerosol precursor on the surface of a piezoelectric transducer may each affect the performance (e.g., the vibratory response) of said piezoelectric transducer in terms of the parameters of the generated aerosol. The physical properties of the aerosol precursor that is in physical contact with the surface of the piezoelectric transducer contribute to the overall mechanical/physical structure of the transducer meaning that variations in the density and volume of the aerosol precursor may (slightly) shift the resonance frequency/frequencies of the piezoelectric transducer. Moreover, changes in the volume of aerosol precursor on the surface of the piezoelectric transducer may change the distance that a cavitation shock must propagate through the aerosol precursor to induce filamentation and consequently aerosolization. As such, changes in both the volume and density of the aerosol precursor can alter the parameters of the generated aerosol.

Similarly, changes in the viscosity of the aerosol precursor affect the fluidic properties of the aerosol precursor, in particular the inertial response of the aerosol precursor to the vibrations of the piezoelectric transducer. This consequently influences the extent (and size) of cavitation within the aerosol precursor, and the propagation of the cavitation shock through the aerosol precursor. As such, changes in the viscosity alter the parameters of the generated aerosol.

The molecular size of the aerosol precursor may correlate with the average droplet size in the generated aerosol.

The one or more parameters of the generated aerosol that may be affected by these parameters of the aerosol precursor may include one or more of: an amount of aerosol precursor entrained in the generated aerosol for each puff of the aerosol generating apparatus in use; an average size of the droplets of aerosol precursor entrained in the generated aerosol; and/or a distribution of the size of the droplets of aerosol precursor entrained in the generated aerosol.

Density, viscosity, and molecular size may be considered to be intrinsic properties of the aerosol precursor. As such, modifying the density, viscosity and molecular size of the aerosol precursor may require modifying the composition of the precursor itself.

In contrast, the volume of the aerosol precursor on the surface of the piezoelectric transducer may be considered to be an extrinsic property of the received aerosol precursor. That is, the amount of aerosol precursor on the surface of the piezoelectric transducer (i.e., the volume of the received aerosol precursor) may be controlled by a user of the aerosol generating apparatuses described herein. Aerosol precursor may be received onto the surface of the piezoelectric transducer to increase the volume of aerosol precursor on the surface of the piezoelectric transducer.

However, it is also possible that, during use of an aerosol generating apparatus, excess aerosol precursor may build up on the surface of the piezoelectric transducer. Such an excess an excess may negatively impact the performance of the piezoelectric transducer and consequently the aerosol generating apparatus.

As such, providing a surface priming signal for the purpose of expelling excess aerosol precursor from the surface of the piezoelectric transducer may improve the performance of the piezoelectric transducer and consequently the aerosol generating apparatus.

The surface priming signal may be a signal that, when the piezoelectric transducer is driven in accordance with the surface priming signal, causes the piezoelectric transducer to vibrate with an amplitude that is sufficient to expel a portion (e.g., the excess portion) of the received aerosol precursor from the surface of the piezoelectric transducer. In this way, driving the piezoelectric transducer with the surface priming signal may facilitate control of one or more parameters of the aerosol generated by the piezoelectric transducer, thereby improving the user experience for a user of the aerosol generating apparatuses described herein.

In some examples, the aerosol precursor may be a liquid aerosol precursor. In other words, the aerosol precursor may be in liquid form. Alternatively, the aerosol precursor may be a gel aerosol precursor - i.e., the aerosol may be in gel form.

In some examples, driving the piezoelectric transducer to expel excess aerosol precursor from the surface of the piezoelectric transducer may further expel excess aerosol precursor from at least a portion of a wick of the aerosol generating apparatus. The wick may be arranged to convey the aerosol precursor from the tank to the piezoelectric transducer.

In addition to excess aerosol precursor building up on the surface of the piezoelectric transducer, when the aerosol precursor is conveyed to the surface of the piezoelectric transducer from the tank via a wick, excess aerosol precursor may build up in the structure of the wick.

The wick may be formed from any material suitable for wicking aerosol precursor from the tank to the surface of the piezoelectric transducer. For example, the wick may be formed from any suitable fibrous material such as, but not limited to, cotton or bamboo fibres.

Expelling excess aerosol precursor from the wick may, in addition to reducing the volume of received aerosol precursor on the surface of the piezoelectric transducer, reduced the amount of aerosol precursor born in the wick and, consequently, reduce the rate of conveyance of aerosol precursor from the tank to the wick. In other words, expelling excess aerosol precursor from the wick may reduce the rate at which aerosol precursor is added to the surface of the piezoelectric transducer.

In some examples, the method may further comprise: providing, to the piezoelectric transducer, a driving signal to drive the piezoelectric transducer so as to generate an aerosol from the received aerosol precursor.

Upon application of a current in a first polarity, opposite faces of the piezoelectric crystal of a piezoelectric transducer respond by expanding, or bulging, outwards to define respective convex surfaces. Conversely, upon application of current in a second polarity opposite to the first polarity, the opposite faces of the piezoelectric crystal of the transducer respond by contracting, or drawing, inwards to define respective concave surfaces. In the context of an aerosol-generating apparatus, driving the piezoelectric transducer in the first polarity may ensure that physical contact between the transducer and the received aerosol precursor can be maintained. Maintaining this physical contact may improve the power efficiency of the inducement of cavitation in the aerosol precursor, and therefore may improve the efficiency of the generation of the aerosol. To this end, in some examples, the driving signal may be a direct current signal to ensure driving of the piezoelectric transducer is carried out in a single polarity.

In some examples, a power level of the surface priming signal may be greater than a power level of the driving signal.

In this way, the driving signal may be suitable for inducing cavitation and one or more shocks in the received aerosol precursor to thereby generate an aerosol without inducing a bulk expulsion of precursor material from the surface of the piezoelectric transducer. Conversely, the surface priming signal may be configured and delivered with a power level sufficient to drive vibrations (or distortions/displacements) in the surface of the piezoelectric transducer that have a large enough energy and amplitude to expel, in bulk, excess aerosol precursor from the surface of the piezoelectric transducer.

In some examples, the method may further comprise: monitoring, while the aerosol generating apparatus is in use, a build-up of aerosol precursor on the surface of the piezoelectric transducer, and providing the surface priming signal to drive the piezoelectric transducer in response to the aerosol precursor crossing a predetermined threshold.

In other words, when an amount of aerosol precursor builds up above a predetermined threshold, the method may involve providing one or more instances of the surface priming signal (e.g., in the form of a boost signal to the driving signal) to expel the excess aerosol precursor from the surface of the piezoelectric transducer so as to maintain a particular (e.g., a preferred or desired) operating condition of the aerosol generating apparatuses described herein.

In this way, the amount of received aerosol precursor may be monitored and controllably maintained to ensure a consistent user experience for a user of the aerosol generating apparatuses described herein.

In some examples, the predetermined threshold may be one or more of: a predetermined volume of aerosol precursor on the surface of the piezoelectric transducer; a predetermined mass of aerosol precursor on the surface of the piezoelectric transducer; a volume of aerosol generated per puff of the aerosol generating apparatus, when in use; and/or a concentration of aerosol generated per puff of the aerosol generating apparatus, when in use.

Both the volume and mass of received aerosol precursor on the surface of the piezoelectric transducer may directly correlate with the amount of received aerosol precursor deposited on the surface of the piezoelectric transducer and, as such, monitoring one or both of the volume and mass of the received aerosol precursor may be a suitable indicator for the amount of build-up of excess aerosol precursor on the surface of the piezoelectric transducer.

Meanwhile, as excess aerosol precursor builds up on the surface of the piezoelectric transducer, the distance that a cavitation shock must propagate through to induce filamentation and consequently aerosolization also increases, thereby reducing the efficiency with which aerosol is generated by the aerosol generating apparatus. As such, a build-up of excess aerosol precursor on the surface of the piezoelectric transducer may reduce one or both of the amount and/or concentration of aerosol generated per puff of the aerosol generating apparatus, when in use. Accordingly, monitoring one or both of the amount and concentration of aerosol generated per puff of the aerosol generating apparatus when in use may be a suitable indicator for the amount of build-up of excess aerosol precursor on the surface of the piezoelectric transducer.

In some examples, the method may further comprise: measuring a response of the piezoelectric transducer to the driving signal; and determining, based on the measured response, a profile of the aerosol precursor.

In this sense, the providing of the surface priming signal may be useable as part of a wider process where the surface priming signal effectively sets (or calibrates) the aerosol generating apparatus to be in a condition from which a profile of the aerosol precursor may be determined.

Determining a profile of the aerosol precursor may be beneficial in a variety of contexts.

As discussed above, the intrinsic properties of an aerosol precursor such as viscosity, density and/or molecular size may also impact one or more parameters of the aerosol generated by the aerosol generating apparatuses described herein. As such, it may be beneficial to determine a profile of the aerosol precursor from which aerosol is being generated so as to modify one or more operational parameters of the aerosol generating apparatus (e.g., a driving frequency, driving power and/or driving duty cycle of the driving signal) to achieve one or more target parameters of the generated aerosol.

The one or more target parameters of the generated aerosol may include one or more of: an amount of aerosol precursor entrained in the generated aerosol for each puff of the aerosol generating apparatus in use; an average size of the droplets of aerosol precursor entrained in the generated aerosol; and/or a distribution of the size of the droplets of aerosol precursor entrained in the generated aerosol.

Additionally or alternatively, determining the profile of the aerosol precursor may be advantageous for determining if the aerosol precursor contained within the tank is formed from an authorised composition and/or if the tank connected to a body of the aerosol generating apparatus is an authorised tank. For example, if the aerosol precursor and/or the tank originates from an unauthorised (e.g., counterfeit) source, use of the unauthorised aerosol precursor and/or unauthorised tank may lead to one or more of: damage to the aerosol generating apparatus (e.g., due to an unsecure or overloaded electrical connection between the unauthorised tank and the body of the aerosol generating apparatus) and/or damage to a user of the aerosol generating apparatus (e.g., because a counterfeit or illicit composition in an aerosol precursor may negatively impact the health of the user).

As such, it is beneficial to be able to verify that the tank and/or aerosol precursor used in the aerosol generating apparatus is an authorised (and known) aerosol precursor and/or tank.

In some examples, determining the profile of the aerosol precursor may involve comparing the measured response of the piezoelectric transducer with one or more reference responses. Each of the one or more reference responses may correspond to a respective authorised aerosol precursor that is authorised for use with the aerosol generating apparatus. Determining the profile of the aerosol precursor may further involve verifying, based on the comparing the measured response with the one or more reference responses, whether the aerosol precursor is an authorised aerosol precursor.

The one or more reference responses may be stored in a database of responses against which a determined profile may be compared e.g., via a look-up function. The database of stored one or more reference responses may be updated on a regular, continuous or as-needed basis Updating the database of one or more reference responses may involve receiving a new one or more reference responses to add into the database from a trusted source - e.g., a remote server maintained by a provider/manufacturer of the aerosol generating apparatuses described herein or any of the components thereof.

The database of one or more reference responses may be stored either on-board the aerosol generating apparatus e.g., in a memory thereof, or in a remote location such as on a remote device or in a remote server. Further, the operation of determining the profile of the aerosol precursor may be performed locally or remotely, either with respect to the aerosol generating apparatus or the database of one or more reference responses. In other words, the determining of the profile of the aerosol precursor may be carried out by a processor or control unit of the aerosol generating apparatus, by a computer that is locally connected to the database of one or more reference responses, or by a computer that is remote from both the aerosol generating apparatus and the database.

In some examples, the method may further comprise, if it is determined that the aerosol precursor is not an authorised aerosol precursor, disabling the aerosol generating apparatus.

In this way, damage to the aerosol generating apparatus and/or to the health of a user of the aerosol generating apparatus may be prevented.

In some examples, the method may further comprise: if it is determined that the aerosol precursor is one of the one or more authorised aerosol precursors, modifying one or more operational parameters of the aerosol generating apparatus according to stored target operational parameters associated with the one or more authorised aerosol precursors.

The one or more operational parameters of the aerosol generating apparatus may include one or more of: a driving frequency, driving power and/or driving duty cycle of a driving signal used to drive the piezoelectric transducer.

The stored target operational parameters may be stored in a database separate from or connected to the database of reference responses. In some examples, the stored target operational parameters may be stored in the database of reference responses in any suitable format - e.g., a key-value structure wherein the target operational parameters associated with a given authorised aerosol precursor are stored as the 'value' in the key-value pair, and the profile/identity of said authorised aerosol precursor is stored as the `key' in the key-value pair.

In some examples, measuring the response of the piezoelectric transducer involves measuring one or more of: the current response of the piezoelectric transducer; the impedance response of the piezoelectric transducer; and/or the voltage response of the piezoelectric transducer to one or more driving signals, each of the one or more driving signals having a different driving frequency.

In some examples, measuring the response of the piezoelectric transducer may involve scanning the driving signal across a range of frequencies and measuring one or more of: the current response of the piezoelectric transducer; the impedance response of the piezoelectric transducer; and/or the voltage response of the piezoelectric transducer across the range of frequencies.

Measuring the electrical response of the piezoelectric transducer may be more accurate and/or reliable than attempting to physically measure the displacements/distortions associated with vibration of the piezoelectric transducer and therefore may facilitate a more precise determination of the profile of the aerosol precursor and a more precise control of the operational parameters of the aerosol generating apparatus.

In another aspect, the present disclosure provides a method of controlling an aerosol generating apparatus. The aerosol generating apparatus includes: a tank for containing an aerosol precursor, a piezoelectric transducer arranged to receive aerosol precursor from the tank and configured to induce cavitation in the received aerosol precursor to thereby generate an aerosol from the received aerosol precursor.

The method comprises: providing to the piezoelectric transducer, a driving signal to drive the piezoelectric transducer so as to generate an aerosol from the received aerosol precursor; measuring a response of the piezoelectric transducer to the driving signal; and determining, based on the measured response, a profile of the aerosol precursor.

The piezoelectric transducer includes a surface of the piezoelectric transducer that receives aerosol precursor from the tank. Upon application of a current through the piezoelectric transducer, the transducer is configured to vibrate, causing displacement/distortion of the surface upon which the aerosol precursor is received. The displacement/distortion of the surface of the piezoelectric transducer induces cavitation in the and a consequent shock in the received aerosol precursor, thereby generating an aerosol. The mechanism for such aerosol generation has been described, for example, in Kooij *et al.* Sci. Rp., 9, 6128 (2019), the entirety of which is incorporated herein by reference.

Upon application of a current in a first polarity, opposite faces of the piezoelectric crystal of a piezoelectric transducer respond by expanding, or bulging, outwards to define respective convex surfaces. Conversely, upon application of current in a second polarity opposite to the first polarity, the opposite faces of the piezoelectric crystal of the transducer respond by contracting, or drawing, inwards to define respective concave surfaces. In the context of an aerosol-generating apparatus, driving the piezoelectric transducer in the first polarity may ensure that physical contact between the transducer and the received aerosol precursor can be maintained. Maintaining this physical contact may improve the power efficiency of the inducement of cavitation in the aerosol precursor, and therefore may improve the efficiency of the generation of the aerosol. To this end, in some examples, the driving signal may be a direct current signal to ensure driving of the piezoelectric transducer is carried out in a single polarity.

Determining a profile of the aerosol precursor may be beneficial in a variety of contexts.

As discussed above, the intrinsic properties of an aerosol precursor such as viscosity, density and/or molecular size may also impact one or more parameters of the aerosol generated by the aerosol generating apparatuses described herein. As such, it may be beneficial to determine a profile of the aerosol precursor from which aerosol is being generated so as to modify one or more operational parameters of the aerosol generating apparatus (e.g., a driving frequency, driving power and/or driving duty cycle of the driving signal) to achieve one or more target parameters of the generated aerosol.

The one or more target parameters of the generated aerosol may include one or more of: an amount of aerosol precursor entrained in the generated aerosol for each puff of the aerosol generating apparatus in use; an average size of the droplets of aerosol precursor entrained in the generated aerosol; and/or a distribution of the size of the droplets of aerosol precursor entrained in the generated aerosol.

Additionally or alternatively, determining the profile of the aerosol precursor may be advantageous for determining if the aerosol precursor contained within the tank is formed from an authorised composition and/or if the tank connected to a body of the aerosol generating apparatus is an authorised tank. For example, if the aerosol precursor and/or the tank originates from an unauthorised (e.g., counterfeit) source, use of the unauthorised aerosol precursor and/or unauthorised tank may lead to one or more of: damage to the aerosol generating apparatus (e.g., due to an unsecure or overloaded electrical connection between the unauthorised tank and the body of the aerosol generating apparatus) and/or damage to a user of the aerosol generating apparatus (e.g., because a counterfeit or illicit composition in an aerosol precursor may negatively impact the health of the user).

As such, it is beneficial to be able to verify that the tank and/or aerosol precursor used in the aerosol generating apparatus is an authorised (and known) aerosol precursor and/or tank.

In some examples, the aerosol precursor may be a liquid aerosol precursor. In other words, the aerosol precursor may be in liquid form. Alternatively, the aerosol precursor may be a gel aerosol precursor - i.e., the aerosol may be in gel form.

In some examples, determining the profile of the aerosol precursor may involve comparing the measured response of the piezoelectric transducer with one or more reference responses. Each of the one or more reference responses may correspond to a respective authorised aerosol precursor that is authorised for use with the aerosol generating apparatus. Determining the profile of the aerosol precursor may further involve verifying, based on the comparing the measured response with the one or more reference responses, whether the aerosol precursor is an authorised aerosol precursor.

The one or more reference responses may be stored in a database of responses against which a determined profile may be compared e.g., via a look-up function. The database of stored one or more reference responses may be updated on a regular, continuous or as needed basis.

Updating the database of one or more reference responses may involve receiving a new one or more reference responses to add into the database from a trusted source - e.g., a remote server maintained by a provider/manufacturer of the aerosol generating apparatuses described herein or any of the components thereof.

The database of one or more reference responses may be stored either on-board the aerosol generating apparatus e.g., in a memory thereof, or in a remote location such as on a remote device or in a remote server. Further, the operation of determining the profile of the aerosol precursor may be performed locally or remotely, either with respect to the aerosol generating apparatus or the database of one or more reference responses. In other words, the determining of the profile of the aerosol precursor may be carried out by a processor or control unit of the aerosol generating apparatus, by a computer that is locally connected to the database of one or more reference responses, or by a computer that is remote from both the aerosol generating apparatus and the database.

In some examples, the method may further comprise: if it is determined that the aerosol precursor is not an authorised aerosol precursor, disabling the aerosol generating apparatus.

In this way, damage to the aerosol generating apparatus and/or to the health of a user of the aerosol generating apparatus may be prevented.

In some examples, the method may further comprise: if it is determined that the aerosol precursor is one of the one or more authorised aerosol precursors, modifying one or more operational parameters of the aerosol generating apparatus according to stored target operational parameters associated with the one of the one or more authorised aerosol precursors.

The one or more operational parameters of the aerosol generating apparatus may include one or more of: a driving frequency, driving power and/or driving duty cycle of a driving signal used to drive the piezoelectric transducer.

The stored target operational parameters may be stored in a database separate from or connected to the database of reference responses. In some examples, the stored target operational parameters may be stored in the database of reference responses in any suitable format - e.g., a key-value structure wherein the target operational parameters associated with a given authorised aerosol precursor are stored as the 'value' in the key-value pair, and the profile/identity of said authorised aerosol precursor is stored as the `key' in the key-value pair.

In some examples, measuring the response of the piezoelectric transducer may involve measuring one or more of: the current response of the piezoelectric transducer; the impedance response of the piezoelectric transducer; and/or the voltage response of the piezoelectric transducer to one or more driving signals, each of the one or more driving signals having a different frequency.

In some examples, measuring the response of the piezoelectric transducer may involve scanning the driving signal across a range of frequencies, and measuring one or more of: the current response of the piezoelectric transducer; the impedance response of the piezoelectric transducer; and/or the voltage response of the piezoelectric transducer across the range of frequencies.

Measuring the electrical response of the piezoelectric transducer may be more accurate and/or reliable than attempting to physically measure the displacements/distortions associated with vibration of the piezoelectric transducer and therefore may facilitate a more precise determination of the profile of the aerosol precursor and a more precise control of the operational parameters of the aerosol generating apparatus.

In some examples, the method may further comprise: providing, to the piezoelectric transducer, a surface priming signal to drive the piezoelectric transducer so as to expel excess aerosol precursor from a surface of the piezoelectric transducer.

The surface priming signal may be a signal that, when the piezoelectric transducer is driven in accordance with the surface priming signal, causes the piezoelectric transducer to vibrate with an amplitude that is sufficient to expel a portion (e.g., the excess portion) of the received aerosol precursor from the surface of the piezoelectric transducer. In this way, driving the piezoelectric transducer with the surface priming signal may facilitate control of one or more parameters of the aerosol generated by the piezoelectric transducer, thereby improving the user experience for a user of the aerosol generating apparatuses described herein.

Providing the surface priming signal may effectively set (or calibrate) the aerosol generating apparatus to be in a condition from which a profile of the aerosol precursor may be determined.

One or more parameters of the aerosol precursor may impact one or more parameters of the aerosol generated by the aerosol generating apparatuses described herein.

The density, viscosity, volume and molecular size of aerosol precursor on the surface of a piezoelectric transducer may each affect the performance (e.g., the vibratory response) of said piezoelectric transducer in terms of the parameters of the generated aerosol. The physical properties of the aerosol precursor that is in physical contact with the surface of the piezoelectric transducer contribute to the overall mechanical/physical structure of the transducer meaning that variations in the density and volume of the aerosol precursor may (slightly) shift the resonance frequency/frequencies of the piezoelectric transducer. Moreover, changes in the volume of aerosol precursor on the surface of the piezoelectric transducer may change the distance that a cavitation shock must propagate through the aerosol precursor to induce filamentation and consequently aerosolization. As such, changes in both the volume and density of the aerosol precursor can alter the parameters of the generated aerosol.

Similarly, changes in the viscosity of the aerosol precursor affect the fluidic properties of the aerosol precursor, in particular the inertial response of the aerosol precursor to the vibrations of the piezoelectric transducer. This consequently influences the extent (and size) of cavitation within the aerosol precursor, and the propagation of the cavitation shock through the aerosol precursor. As such, changes in the viscosity alter the parameters of the generated aerosol.

The molecular size of the aerosol precursor may correlate with the average droplet size in the generated aerosol.

The one or more parameters of the generated aerosol that may be affected by these parameters of the aerosol precursor may include one or more of: an amount of aerosol precursor entrained in the generated aerosol for each puff of the aerosol generating apparatus in use; an average size of the droplets of aerosol precursor entrained in the generated aerosol; and/or a distribution of the size of the droplets of aerosol precursor entrained in the generated aerosol.

Density, viscosity, and molecular size may be considered to be intrinsic properties of the aerosol precursor. As such, modifying the density, viscosity and molecular size of the aerosol precursor may require modifying the composition of the precursor itself.

In contrast, the volume of the aerosol precursor on the surface of the piezoelectric transducer may be considered to be an extrinsic property of the received aerosol precursor. That is, the amount of aerosol precursor on the surface of the piezoelectric transducer (i.e., the volume of the received aerosol precursor) may be controlled by a user of the aerosol generating apparatuses described herein. Aerosol precursor may be received onto the surface of the piezoelectric transducer to increase the volume of aerosol precursor on the surface of the piezoelectric transducer.

However, it is also possible that, during use of an aerosol generating apparatus, excess aerosol precursor may build up on the surface of the piezoelectric transducer. Such an excess an excess may negatively impact the performance of the piezoelectric transducer and consequently the aerosol generating apparatus.

As such, providing a surface priming signal for the purpose of expelling excess aerosol precursor from the surface of the piezoelectric transducer may improve the performance of the piezoelectric transducer and consequently the aerosol generating apparatus.

In some examples, the providing the surface priming signal may be carried out before the measuring the response of the piezoelectric transducer to the driving signal.

In this way, it can be ensured that providing the surface priming signal effectively sets (or calibrates) the aerosol generating apparatus to be in a condition from which a profile of the aerosol precursor may be determined.

In some examples, driving the piezoelectric transducer according to the surface priming signal to expel excess aerosol precursor form the surface of the piezoelectric transducer may further expel excess aerosol precursor from at least a portion of a wick of the aerosol generating apparatus. The wick may be arranged to convey the aerosol precursor from the tank to the piezoelectric transducer.

In addition to excess aerosol precursor building up on the surface of the piezoelectric transducer, when the aerosol precursor is conveyed to the surface of the piezoelectric transducer from the tank via a wick, excess aerosol precursor may build up in the structure of the wick.

The wick may be formed from any material suitable for wicking aerosol precursor from the tank to the surface of the piezoelectric transducer. For example, the wick may be formed from any suitable fibrous material such as, but not limited to, cotton or bamboo fibres.

Expelling excess aerosol precursor from the wick may, in addition to reducing the volume of received aerosol precursor on the surface of the piezoelectric transducer, reduced the amount of aerosol precursor born in the wick and, consequently, reduce the rate of conveyance of aerosol precursor from the tank to the wick. In other words, expelling excess aerosol precursor from the wick may reduce the rate at which aerosol precursor is added to the surface of the piezoelectric transducer.

In some examples, a power level of the surface priming signal may be greater than a power level of the driving signal.

In this way, the driving signal may be suitable for inducing cavitation and one or more shocks in the received aerosol precursor to thereby generate an aerosol without inducing a bulk expulsion of precursor material from the surface of the piezoelectric transducer. Conversely, the surface priming signal may be configured and delivered with a power level sufficient to drive vibrations (or distortions/displacements) in the surface of the piezoelectric transducer that have a large enough energy and amplitude to expel, in bulk, excess aerosol precursor from the surface of the piezoelectric transducer.

In some examples, the method may further comprise: monitoring, while the aerosol generating apparatus is in use, a build-up of aerosol precursor on the surface of the piezoelectric transducer, and providing the surface priming signal to drive the piezoelectric transducer in response to the aerosol precursor crossing a predetermined threshold.

In other words, when an amount of aerosol precursor builds up above a predetermined threshold, the method may involve providing one or more instances of the surface priming signal (e.g., in the form of a boost signal to the driving signal) to expel the excess aerosol precursor from the surface of the piezoelectric transducer so as to maintain a particular (e.g., a preferred or desired) operating condition of the aerosol generating apparatuses described herein.

In this way, the amount of received aerosol precursor may be monitored and controllably maintained to ensure a consistent user experience for a user of the aerosol generating apparatuses described herein.

In some examples, the predetermined threshold may be one or more of: a predetermined volume of aerosol precursor on the surface of the piezoelectric transducer; a predetermined mass of aerosol precursor on the surface of the piezoelectric transducer; a volume of aerosol generated per puff of the aerosol generating apparatus, when in use; and/or a concentration of aerosol generated per puff of the aerosol generating apparatus, when in use.

Both the volume and mass of received aerosol precursor on the surface of the piezoelectric transducer may directly correlate with the amount of received aerosol precursor deposited on the surface of the piezoelectric transducer and, as such, monitoring one or both of the volume and mass of the received aerosol precursor may be a suitable indicator for the amount of build-up of excess aerosol precursor on the surface of the piezoelectric transducer.

Meanwhile, as excess aerosol precursor builds up on the surface of the piezoelectric transducer, the distance that a cavitation shock must propagate through to induce filamentation and consequently aerosolization also increases, thereby reducing the efficiency with which aerosol is generated by the aerosol generating apparatus. As such, a build-up of excess aerosol precursor on the surface of the piezoelectric transducer may reduce one or both of the amount and/or concentration of aerosol generated per puff of the aerosol generating apparatus, when in use. Accordingly, monitoring one or both of the amount and concentration of aerosol generated per puff of the aerosol generating apparatus when in use may be a suitable indicator for the amount of build-up of excess aerosol precursor on the surface of the piezoelectric transducer.

In another aspect, the present disclosure provides an aerosol generating system comprising: a tank for containing an aerosol precursor; and a piezoelectric transducer arranged to receive aerosol precursor from the tank on a surface of the piezoelectric transducer; and electrical circuitry. The piezoelectric transducer is configured to induce cavitation in the received aerosol precursor to thereby generate an aerosol from the received aerosol precursor. The electrical circuitry is configured to carry out any of the methods described herein.

The tank and piezoelectric transducer may be embodied in an aerosol generating apparatus of the aerosol generating system.

The electrical circuitry may be embodied in the same aerosol generating apparatus of the aerosol generating system or in an external device of the aerosol generating system separate from the aerosol generating apparatus.

In another aspect, the present disclosure provides a computer-readable medium comprising instructions that, when carried out by a computer, cause the computer to carry out any of the methods described herein.

The computer may, for example, be a control unit of any of the aerosol generating apparatuses described herein.

The computer may, for example, be a remote computer that is communicatively connectable to any of the aerosol generating apparatuses described herein.

The computer may, for example, be embodied as a distributed computing system including, for example, a control unit of any of the aerosol generating apparatuses described herein and a remote computer that is communicatively connectable to any of the aerosol generating apparatuses described herein, e.g., via a communications interface of the aerosol generating apparatus.

In another aspect, the present disclosure provides electrical circuitry for an aerosol generating system, the electrical circuitry being arranged to perform any of the methods described herein.

In embodiments, the electrical circuitry is implemented as one or more processors, which are configured to implement the disclosed steps, e.g. as the controller. The processors may execute program code stored on electronic memory and/or may execute logic, e.g. as a logic array, gate array, structured gate array.

As will be apparent from the present disclosure, the methods described herein may be carried out, or implemented, by a computer. The computer may, for example, be a processor installed in the aerosol generating apparatus and configured to operate as a control unity of the aerosol generating apparatus. Alternatively, the computer may, for example, be a remote computer communicatively connectable to the aerosol generating apparatus via a communications interface of the aerosol generating apparatus. Alternatively, the computer may be embodied as a distributed computing environment, including for example, both a control unit installed in the aerosol generating apparatus and a remote computer that is communicatively connectable to the control unit via a communications interface of the aerosol generating apparatus.

Moreover, the acts described herein may be embodied using computer-executable instructions that can be implemented by one or more processors and/or stored on a computer-readable medium or media. The computer-executable instructions can include routines, sub-routines; programs; threads of execution, and/or the like. Still further, results of acts of the methods can be stored in a computer-readable medium, displayed on a display device, and/or the like.

The order of the operations of the methods described herein is exemplary, but the steps may be carried out in any suitable order, or simultaneously where appropriate. Additionally, steps may be added or substituted in, or individual steps may be deleted from any of the methods without departing from the scope of the subject matter described herein. Aspects of any of the examples described above may be combined with aspects of any of the other examples described to form further examples without losing the effect sought.

Various functions described herein can be implemented in hardware, software, or any combination thereof. If implemented in software, the functions can be stored on or transmitted over as one or more instructions or code on a computer-readable medium. Computer-readable media may include, for example, computer-readable storage media. Computer-readable storage media may include volatile or non-volatile, removable or non-removable media implemented in any method or technology for storage of information such as computer readable instructions, data structures, program modules or other data. A computer-readable storage media can be any available storage media that may be accessed by a computer. By way of example, and not limitation, such computer-readable storage media may comprise RAM, ROM, EEPROM, flash memory or other memory devices, CD-ROM or other optical disc storage, magnetic disc storage or other magnetic storage devices, or any other medium that can be used to carry or store desired program code in the form of instructions or data structures and that can be accessed by a computer.

Although illustrated as a local device it will be appreciated that the computing device may be located remotely and accessed via a network or other communication link (for example using a communication interface).

The term 'computer' is used herein to refer to any device with processing capability such that it can execute instructions. Those skilled in the art will realise that such processing capabilities are incorporated into many different devices and therefore the term 'computer' includes PCs, servers, mobile telephones, personal digital assistants and many other devices.

Those skilled in the art will realise that storage devices utilised to store program instructions can be distributed across a network. For example, a remote computer may store an example of the process described as software. A local or terminal computer may access the remote computer and download a part or all of the software to run the program. Alternatively, the local computer may download pieces of the software as needed or execute some software instructions at the local terminal and some at the remote computer (or computer network). Those skilled in the art will also realise that by utilising conventional techniques known to those skilled in the art that all, or a portion of the software instructions may be carried out by a dedicated circuit, such as a DSP, programmable logic array, or the like.

It will be understood that the benefits and advantages described above may relate to one embodiment or may relate to several embodiments. The embodiments are not limited to those that solve any or all the stated problems or those that have any or all of the stated benefits and advantages. Variants should be considered to be included into the scope of the invention.

The preceding summary is provided for purposes of summarizing some examples to provide a basic understanding of aspects of the subject matter described herein. Accordingly, the above-described features should not be construed to narrow the scope or spirit of the subject matter described herein in any way. Moreover, the above and/or proceeding examples may be combined in any suitable combination to provide further examples, except where such a combination is clearly impermissible or expressly avoided. Other features, aspects, and advantages of the subject matter described herein will become apparent from the following text and the accompanying drawings.

### BRIEF DESCRIPTION OF THE FIGURES

Aspects, features and advantages of the present disclosure will become apparent from the following description of examples in reference to the appended drawings in which like numerals denote like elements.
Fig. 1 is a block system diagram showing an example aerosol generating apparatus.
Fig. 2 is a block system diagram showing an example implementation of the apparatus of Fig. 1, where the aerosol generating apparatus is configured to generate aerosol from a liquid precursor.
Figs. 3A and 3B are schematic diagrams showing an example implementation of the apparatus of Fig. 2.
Fig. 4 is a block system diagram showing an example system for managing an aerosol generating apparatus.
Fig. 5 shows an example of a circuit for modelling the behaviour of an exemplary piezoelectric transducer.
Fig. 6 shows a portion of an exemplary driving circuit using an H-bridge.
Fig. 7 shows an example of an improved driving circuit for driving a piezoelectric transducer.
Fig. 8 shows an exemplary frequency response in the current flowing through a piezoelectric transducer.
Fig. 9 shows a method of controlling an aerosol generating apparatus.
Fig. 10 shows a method of controlling an aerosol generating apparatus.

### DETAILED DESCRIPTION OF EMBODIMENTS

Before describing several examples implementing the present disclosure, it is to be understood that the present disclosure is not limited by specific construction details or process steps set forth in the following description and accompanying drawings. Rather, it will be apparent to those skilled in the art having the benefit of the present disclosure that the systems, apparatuses and/or methods described herein could be embodied differently and/or be practiced or carried out in various alternative ways.

Unless otherwise defined herein, scientific and technical terms used in connection with the presently disclosed inventive concept(s) shall have the meanings that are commonly understood by those of ordinary skill in the art, and known techniques and procedures may be performed according to conventional methods well known in the art and as described in various general and more specific references that may be cited and discussed in the present specification.

Any patents, published patent applications, and non-patent publications mentioned in the specification are hereby incorporated by reference in their entirety.

All examples implementing the present disclosure can be made and executed without undue experimentation in light of the present disclosure. While particular examples have been described, it will be apparent to those of skill in the art that variations may be applied to the systems, apparatus, and/or methods and in the steps or in the sequence of steps of the methods described herein without departing from the concept, spirit, and scope of the inventive concept(s). All such similar substitutions and modifications apparent to those skilled in the art are deemed to be within the spirit, scope, and concept of the inventive concept(s) as defined by the appended claims.

The use of the term "a" or "an" in the claims and/or the specification may mean "one," as well as "one or more," "at least one," and "one or more than one." As such, the terms "a," "an," and "the," as well as all singular terms, include plural referents unless the context clearly indicates otherwise. Likewise, plural terms shall include the singular unless otherwise required by context.

The use of the term "or" in the present disclosure (including the claims) is used to mean an inclusive "and/or" unless explicitly indicated to refer to alternatives only or unless the alternatives are mutually exclusive. For example, a condition "A or B" is satisfied by any of the following: A is true (or present) and B is false (or not present), A is false (or not present) and B is true (or present), and both A and B are true (or present).

As used in this specification and claim(s), the words "comprising, "having," "including," or "containing" (and any forms thereof, such as "comprise" and "comprises," "have" and "has," "includes" and "include," or "contains" and "contain," respectively) are inclusive or open-ended and do not exclude additional, unrecited elements or method steps.

Unless otherwise explicitly stated as incompatible, or the physics or otherwise of the embodiments, examples, or claims prevent such a combination, the features of examples disclosed herein, and of the claims, may be integrated together in any suitable arrangement, especially ones where there is a beneficial effect in doing so. This is not limited to only any specified benefit, and instead may arise from an "ex post facto" benefit. This is to say that the combination of features is not limited by the described forms, particularly the form (e.g. numbering) of example(s), embodiment(s), or dependency of claim(s).

Moreover, this also applies to the phrase "in one embodiment," "according to an embodiment," and the like, which are merely a stylistic form of wording and are not to be construed as limiting the following features to a separate embodiment to all other instances of the same or similar wording. This is to say, a reference to 'an,' 'one,' or 'some' embodiment(s) may be a reference to any one or more, and/or all embodiments, or combination(s) thereof, disclosed. Also, similarly, the reference to "the" embodiment may not be limited to the immediately preceding embodiment. Further, all references to one or more embodiments or examples are to be construed as non-limiting to the claims.

The present disclosure may be better understood in view of the following explanations, wherein the terms used that are separated by "or" may be used interchangeably:
As used herein, an "aerosol generating apparatus" (or "electronic(e)-cigarette") may be an apparatus configured to deliver an aerosol to a user for inhalation by the user. The apparatus may additionally/alternatively be referred to as a "smoking substitute apparatus", if it is intended to be used instead of a conventional combustible smoking article. As used herein a combustible "smoking article" may refer to a cigarette, cigar, pipe or other article, that produces smoke (an aerosol comprising solid particulates and gas) via heating above the thermal decomposition temperature (typically by combustion and/or pyrolysis). An aerosol generated by the apparatus may comprise an aerosol with particle sizes of 0.2 - 7 microns, 2-3 microns, or less than 10 microns, or less than 7 microns, or less than 3 microns, or less than 2 microns. This particle size may be achieved by control of one or more of: driving parameters of the ultrasonic generator; flow properties including turbulence and velocity. The generation of aerosol by the aerosol generating apparatus may be controlled by an input device. The input device may be configured to be user-activated and may for example include or take the form of an actuator (e.g. actuation button) and/or an airflow sensor.

Each occurrence of the aerosol generating apparatus being caused to generate aerosol for a period of time (which may be variable) may be referred to as an "activation" of the aerosol generating apparatus. The aerosol generating apparatus may be arranged to allow an amount of aerosol delivered to a user to be varied per activation (as opposed to delivering a fixed dose of aerosol), e.g. by activating an aerosol generating unit of the apparatus for a variable amount of time, e.g. based on the strength/duration of a draw of a user through a flow path of the apparatus (to replicate an effect of smoking a conventional combustible smoking article).

The aerosol generating apparatus may be portable. As used herein, the term "portable" may refer to the apparatus being for use when held by a user.

As used herein, an "aerosol generating system" may be a system that includes an aerosol generating apparatus and optionally other circuitry/components associated with the function of the apparatus, e.g. one or more external devices and/or one or more external components (here "external" is intended to mean external to the aerosol generating apparatus). As used herein, an "external device" and "external component" may include one or more of a: a charging device, a mobile device (which may be connected to the aerosol generating apparatus, e.g. via a wireless or wired connection); a networked-based computer (e.g. a remote server); a cloud-based computer; any other server system.

An example aerosol generating system may be a system for managing an aerosol generating apparatus. Such a system may include, for example, a mobile device, a network server, as well as the aerosol generating apparatus.

As used herein, an "aerosol" may include a suspension of liquid droplets of precursor. An aerosol may include one or more components of the precursor.

As used herein, a "precursor" may include one or more of a: liquid; gel. The precursor may be processed by an aerosol generating unit of an aerosol generating apparatus to generate an aerosol. The precursor may include one or more of: an active component; a carrier; a flavouring. The active component may include one or more of nicotine; caffeine; a cannabidiol oil; a non-pharmaceutical formulation, e.g. a formulation which is not for treatment of a disease or physiological malfunction of the human body. The active component may be carried by the carrier, which may be a liquid, including propylene glycol and/or glycerine. The term "flavouring" may refer to a component that provides a taste and/or a smell to the user. The flavouring may include one or more of: Ethylvanillin (vanilla); menthol, Isoamyl acetate (banana oil); or other. The precursor may include a carrier; a flavouring.

As used herein, a "storage portion" may be a portion of the apparatus adapted to store the precursor. It may be implemented as fluid-holding reservoir depending on the implementation of the precursor as defined above.

As used herein, a "flow path" may refer to a path or enclosed passageway through an aerosol generating apparatus, e.g. for delivery of an aerosol to a user. The flow path may be arranged to receive aerosol from an aerosol generating unit. When referring to the flow path, upstream and downstream may be defined in respect of a direction of flow in the flow path, e.g. with an outlet being downstream of an inlet.

As used herein, a "delivery system" may be a system operative to deliver an aerosol to a user. The delivery system may include a mouthpiece and a flow path. The delivery system may be at least partly within the aerosol generating component.

As used herein, a "flow" may refer to a flow in a flow path. A flow may include aerosol generated from the precursor. The flow may include air, which may be induced into the flow path via a puff by a user.

As used herein, a "puff" (or "inhale" or "draw") by a user may refer to expansion of lungs and/or oral cavity of a user to create a pressure reduction that induces flow through the flow path.

As used herein, an "aerosol generating unit" may refer to a device configured to generate an aerosol from a precursor. The aerosol generating unit may include a unit to generate an aerosol directly from the precursor (e.g. an atomiser including an ultrasonic system). A plurality of aerosol generating units to generate a plurality of aerosols (for example, from a plurality of different aerosol precursors) may be present in an aerosol generating apparatus.

As used herein, an "ultrasonic generator" may refer to a piezoelectric transducer capable of vibrating at ultrasonic frequencies, i.e., at frequencies greater than 20kHz. In some examples, the piezoelectric transducer may be capable of vibrating at even higher frequencies, e.g., at frequencies of 100 kHz or above, 500 kHz or above, 1 MHz or more, 2 MHz or more, 5 MHz or more, or 10 MHz or more. The piezoelectric transducer may be adapted to vibrate in response to a driving signal, and in particular adapted to vibrate at the frequency of the driving signal. The driving signal may be generated, for example, using direct digital synthesis or any other suitable method.

As used herein, a "piezoelectric transducer" may refer to an ultrasonic transducer comprising a piezoelectric crystal, which generates a mechanical strain internally in response to an electric field. A rapidly changing electric field, such as an ultrasonic frequency driving signal, results in rapidly changing mechanical strain within the piezoelectric crystal causing it to vibrate. The piezoelectric transducer will have an aerosolization surface from which the aerosol is generated. The aerosolization surface typically faces into an aerosolization chamber.

As used herein, an **"aerosol generating component"** may refer to a component that includes an aerosol precursor. The component may include an aerosol generating unit e.g. it may be arranged as a cartomizer. The component may include a mouthpiece. The component may include an information carrying medium. The component may include a storage portion, e.g. a reservoir or tank, for storage of the aerosol precursor.

With liquid or gel implementations of the aerosol precursor, e.g. an e-liquid, the component may be referred to as a "capsule" or a "pod" or an "e-liquid consumable." In some embodiments, the aerosol precursor component may be affixed to the device body to form the aerosol generating apparatus. In these embodiments, the reservoir/tank may be refillable.

The aerosol generating component e.g. the capsule, pod, or consumable may be for releasable coupling to a device body to form the aerosol generating apparatus.

The device body may comprise a power supply for powering the aerosol generating unit.

As used herein, an "information carrying medium" may include one or more arrangements for storage of information on any suitable medium. Examples include: a computer readable medium; a Radio Frequency Identification (RFID) transponder; codes encoding information, such as optical (e.g. a bar code or QR code) or mechanically read codes (e.g. a configuration of the absence or presents of cutouts to encode a bit, through which pins or a reader may be inserted).

As used herein, "electrical circuitry" may refer to one or more electrical components, examples of which may include: an Application Specific Integrated Circuit (ASIC) or other programmable logic; electronic/electrical componentry (which may include combinations of transistors, resistors, capacitors, inductors etc); one or more processors (e.g., the circuitry structure of the processor); a non-transitory memory (e.g. implemented by one or more memory devices), that may store one or more software or firmware programs; a combinational logic circuit; interconnection of the aforesaid. The electrical circuitry may be located entirely at the apparatus, or distributed between the apparatus and/or on one or more external devices in communication with the apparatus, e.g. as part of a system. The electrical circuitry may include a controller or control unit.

As used herein, a "processing resource" (or "processor" or "controller") may refer to one or more units for processing data, examples of which may include an ASIC, microcontroller, FPGA, microprocessor, digital signal processor (DSP) capability, state machine or other suitable component. A processing resource may be configured to execute a computer program, e.g. which may take the form of machine-readable instructions, which may be stored on a non-transitory memory and/or programmable logic. The processing resource may have various arrangements corresponding to those discussed for the circuitry, e.g. on-board and/or off board the apparatus as part of the system. As used herein, any machine executable instructions, or computer readable media, may be configured to cause a disclosed method to be carried out, e.g. by an aerosol generating apparatus or system as disclosed herein, and may therefore be used synonymously with the term method.

As used herein, an "external device" (or "peripheral device") may include one or more electronic components external to an aerosol generating apparatus. Those components may be arranged at the same location as the aerosol generating apparatus or remote from the apparatus. An external device may comprise electronic computer devices including: a smartphone; a PDA; a video game controller; a tablet; a laptop; or other like device.

As used herein, a "computer readable medium/media" (or "memory" or "data storage") may include any medium capable of storing a computer program, and may take the form of any conventional non-transitory memory, for example one or more of: random access memory (RAM); a CD; a hard drive; a solid state drive; a memory card; a DVD. The memory may have various arrangements corresponding to those discussed for the circuitry /processor. The present disclosure includes a computer readable medium configured to cause an apparatus or system disclosed herein to perform a method as disclosed herein.

As used herein, a "communication resource" (or "communication interface") may refer to hardware and/or firmware for electronic information/data transfer. The communication resource may be configured for wired communication ("wired communication resources") or wireless communication ("wireless communication resource"). Wireless communication resources may include hardware to transmit and receive signals by radio and may include various protocol implementations e.g. the 802.11 standard described in the Institute of Electronics Engineers (IEEE) and Bluetooth^{™} from the Bluetooth Special Interest Group of Kirkland Wash. Wired communication resources may include; Universal Serial Bus (USB); High-Definition Multimedia Interface (HDMI) or other protocol implementations. The apparatus may include communication resources for wired or wireless communication with an external device.

As used herein, a "network" (or "computer network") may refer to a system for electronic information/data transfer between a plurality of apparatuses/devices. The network may, for example, include one or more networks of any type, which may include: a Public Land Mobile Network (PLMN); a telephone network (e.g. a Public Switched Telephone Network (PSTN) and/or a wireless network); a local area network (LAN); a metropolitan area network (MAN); a wide area network (WAN); an Internet Protocol Multimedia Subsystem (IMS) network; a private network; the Internet; an intranet.

It will be appreciated that any of the disclosed methods (or corresponding apparatuses, programs, data carriers, etc.) may be carried out by either a host or client, depending on the specific implementation (i.e. the disclosed methods/apparatuses are a form of communication(s), and as such, may be carried out from either 'point of view', i.e. in corresponding to each other fashion). Furthermore, it will be understood that the terms "receiving" and "transmitting" encompass "inputting" and "outputting" and are not limited to an RF context of transmitting and receiving electromagnetic (e.g. radio) waves. Therefore, for example, a chip or other device or component for realizing embodiments could generate data for output to another chip, device or component, or have as an input data from another chip, device, or component, and such an output or input could be referred to as "transmit" and "receive" including gerund forms, that is, "transmitting" and "receiving," as well as such "transmitting" and "receiving" within an RF context.

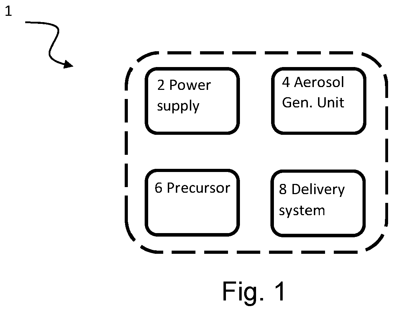

Referring to Fig. 1, an example aerosol generating apparatus 1 includes a power supply 2, for supply of electrical energy. The apparatus 1 includes an aerosol generating unit 4 that is driven by the power supply 2. The power supply 2 may include an electric power supply in the form of a battery and/or an electrical connection to an external power source. The apparatus 1 includes a precursor 6, which in use is aerosolised by the aerosol generating unit 4 to generate an aerosol. The aerosol generating unit 4 includes a piezoelectric transducer (discussed below) configured to induce, by vibration of the piezoelectric transducer i.e. vibration of an aerosolization surface of the piezoelectric transducer, cavitation in the precursor 6. Collapse of the cavities in the precursor 6 induces a shock that propagates through the liquid precursor 6. This shock disturbs a surface of the liquid precursor 6 that interfaces with air within an aerosolization chamber of the aerosol generating apparatus 1 (which in turn is in fluid communication with an airflow path within the aerosol generating apparatus). These disturbances take the form of ripples, also known as capillary waves, that form ligaments at the peaks of the ripples/waves, pinch off and expel droplets from the liquid precursor 6 into the airflow path, thereby aerosolising the precursor 6 to generate the aerosol. The apparatus 2 includes a delivery system 8 for delivery of the aerosol to a user. Although described herein as a liquid precursor 6, the precursor may also take an alternative form e.g., a gel-based aerosol precursor.

Electrical circuitry (not shown in figure 1) may be implemented to control the interoperability of the power supply 2 and aerosol generating unit 4.

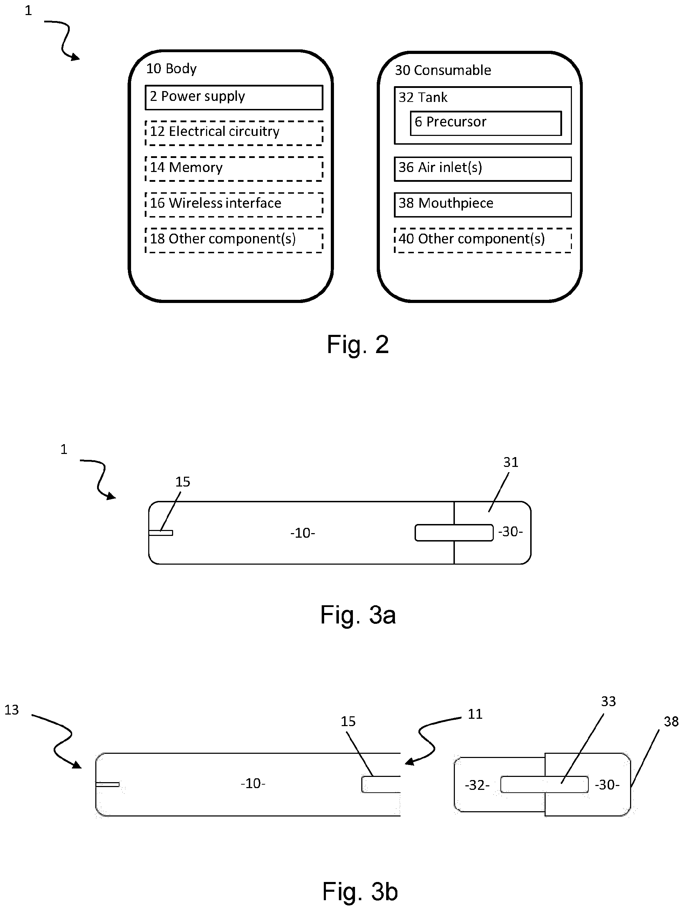

Fig. 2 shows an implementation of the apparatus 1 of Fig. 1, where the aerosol generating apparatus 1 is configured to generate aerosol from a liquid precursor.

In this example, the apparatus 1 includes a device body 10 and a consumable 30.

In this example, the body 10 includes the power supply 2. The body may additionally include any one or more of electrical circuitry 12, a memory 14, a wireless interface 16, one or more other components 18.

The electrical circuitry 12 may include a processing resource for controlling one or more operations of the body 10 and consumable 30, e.g. based on instructions stored in the memory 14.

The wireless interface 16 may be configured to communicate wirelessly with an external (e.g. mobile) device, e.g. via Bluetooth, Bluetooth LE (Low Energy), or WiFi.

The other component(s) 18 may include one or more user interface devices configured to convey information to a user and/or a charging port, for example (see e.g. Fig. 3).

The consumable 30 includes a storage portion implemented here as a tank 32 which stores the liquid precursor 6 (e.g. e-liquid). The consumable 30 also includes one or more air inlets 36, and a mouthpiece 38. The consumable 30 may include one or more other components 40.

The body 10 and consumable 30 may each include a respective electrical interface (not shown) to provide an electrical connection between one or more components of the body 10 with one or more components of the consumable 30. In this way, electrical power can be supplied to components of the consumable 30, without the consumable 30 needing to have its own power supply.

The piezoelectric transducer 34 of the aerosol generating unit 4 is arranged to be in electrical contact with one or more components of the body 10. For example, the power supply 2 may be configured to provide power to the piezoelectric transducer 34. Additionally or alternatively, the piezoelectric transducer 34 may be in electrical contact/communication with one or more of the electrical circuitry 12, memory 14, wireless interface 16 or one or more of the one or more other components 18 e.g., to receive instructions to adjust an operating parameter of the piezoelectric transducer 34 and/or to transmit data indicative of the operational parameters of the piezoelectric transducer 34.

Moreover, the piezoelectric transducer 34 is arranged to be in fluid communication with the tank 32 e.g. via a wick such that the liquid precursor can be provided to the aerosolization surface of the piezoelectric transducer 34.

In use, a user may activate the aerosol generating apparatus 1 when inhaling through the mouthpiece 38, i.e. when performing a puff. The puff, performed by the user, may initiate a flow through a flow path in the consumable 30 which extends from the air inlet(s) 36 to the mouthpiece 38 via a region (i.e. an aerosolization chamber) in proximity to the piezoelectric transducer 34.

Activation of the aerosol generating apparatus 1 may be initiated, for example, by an airflow sensor in the body 10 which detects airflow in the aerosol generating apparatus 1 (e.g. caused by a user inhaling through the mouthpiece), or by actuation of an actuator included in the body 10. Upon activation, the electrical circuitry 12 (e.g. under control of the processing resource) may supply electrical energy from the power supply 2 to the piezoelectric transducer 34 of the aerosol generating unit 4, which may cause the piezoelectric transducer 34 to induce cavitation in the liquid precursor 6 drawn from the tank so as to produce an aerosol which is carried by the flow out of the mouthpiece 38.

In some examples, the consumable may include a wick, wherein a first portion of the wick extends into the tank 32 in order to draw liquid precursor 6 out from the tank 32 and wherein a second portion of the wick is arranged to convey the drawn liquid precursor 6 to the aerosolization surface piezoelectric transducer 34of the aerosol generating unit 4.

In this example, the delivery system 8 is provided by the above-described flow path and mouthpiece 38.

In variant embodiments (not shown), any one or more of the precursor 6, air inlet(s) 36 and mouthpiece 38, may be included in the body 10. For example, the mouthpiece 36 may be included in the body 10 with the precursor 6 arranged as a separable cartomizer.

Figs. 3A and 3B show an example implementation of the aerosol generating apparatus 1 of Fig. 2. In this example, the consumable 30 is implemented as a capsule/pod, which is shown in Fig. 3A as being physically coupled to the body 10, and is shown in Fig. 3B as being decoupled from the body 10.

In this example, the body 10 and the consumable 30 are configured to be physically coupled together by pushing the consumable 30 into an aperture in a top end 11 the body 10, with the consumable 30 being retained in the aperture via an interference fit.

In other examples (not shown), the body 10 and the consumable 30 could be physically coupled together in other ways, e.g. by screwing one onto the other, through a bayonet fitting, or through a snap engagement mechanism, for example.

The body 10 also includes a charging port (not shown) at a bottom end 13 of the body 10.

The body 10 also includes a user interface device configured to convey information to a user. Here, the user interface device is implemented as a light 15, which may e.g. be configured to illuminate when the apparatus 1 is activated. Other user interface devices are possible, e.g. to convey information haptically or audibly to a user.

In this example, the consumable 30 has an opaque cap 31, a translucent tank 32 and a translucent window 33. When the consumable 30 is physically coupled to the body 10 as shown in Fig. 3A, only the cap 31 and window 33 can be seen, with the tank 32 being obscured from view by the body 10. The body 10 includes a slot 15 to accommodate the window 33. The window 33 is configured to allow the amount of liquid precursor 6 in the tank 32 to be visually assessed, even when the consumable 30 is physically coupled to the body 10.

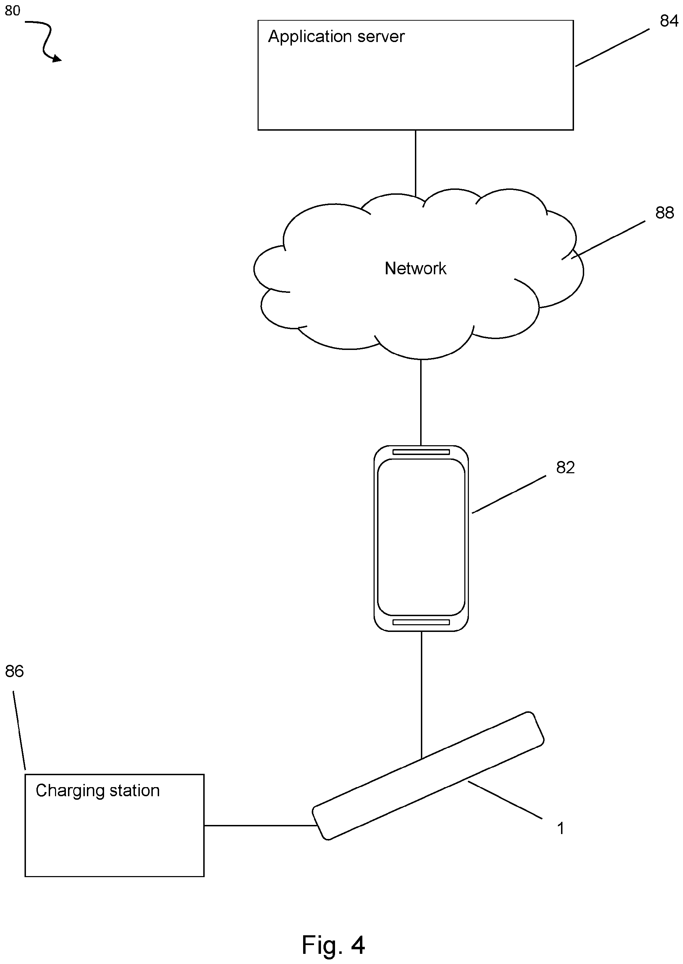

Fig. 4 shows an example system 80 for managing an aerosol generating apparatus 1, such as those described above with reference to any of Figs. 1-3B.

The system 80 as shown in Fig. 1 includes a mobile device 82, an application server 84, an optional charging station 86, as well as the aerosol generating apparatus 1.

In this example, aerosol generating apparatus 1 is configured to communicate wirelessly, e.g. via Bluetooth^{™}, with an application (or "app") installed on the mobile device 2, via a wireless interface included in the aerosol generating apparatus 1 and via a wireless interface included in the mobile device 82. The mobile device 82 may be a mobile phone, for example. The application on the mobile phone is configured to communicate with the application server 84, via a network 88. The application server 84 may utilise cloud storage, for example.

The network 88 may include a cellular network and/or the internet.

In other examples, the aerosol generating apparatus 1 may be configured to communicate with the application server 84 via a connection that does not involve the mobile device 82, e.g. via a narrowband internet of things ("NB-loT") or satellite connection. In some examples, the mobile device 82 may be omitted from the system 80.

A skilled person would readily appreciate that the mobile device 82 may be configured to communicate via the network 88 according to various communication channels, preferably a wireless communication channel such as via a cellular network (e.g. according to a standard protocol, such as 3G or 4G) or via a Wi-Fi network.

The app installed on the mobile device 82 and the application server 84 may be configured to assist a user with managing their aerosol generating apparatus 1, based on information communicated between the aerosol generating apparatus 1 and the app, information communicated directly between the aerosol generating apparatus 1 and the application server 84, and/or information communicated between the app and the application server 84.

The charging station 86 (if present) may be configured to charge (and optionally communicate with) the aerosol generating apparatus 1, via a charging port on the aerosol generating apparatus 1. The charging port on the smoking substitute device 10 may be a USB port, for example, which may allow the aerosol generating apparatus 1 to be charged by any USB-compatible device capable of delivering power to the aerosol generating apparatus 1 via a suitable USB cable (in this case the USB-compatible device would be acting as the charging station 86). Alternatively, the charging station could be a docking station specifically configured to dock with the aerosol generating apparatus 1 and charge the aerosol generating apparatus 1via the charging port on the aerosol generating apparatus 1.

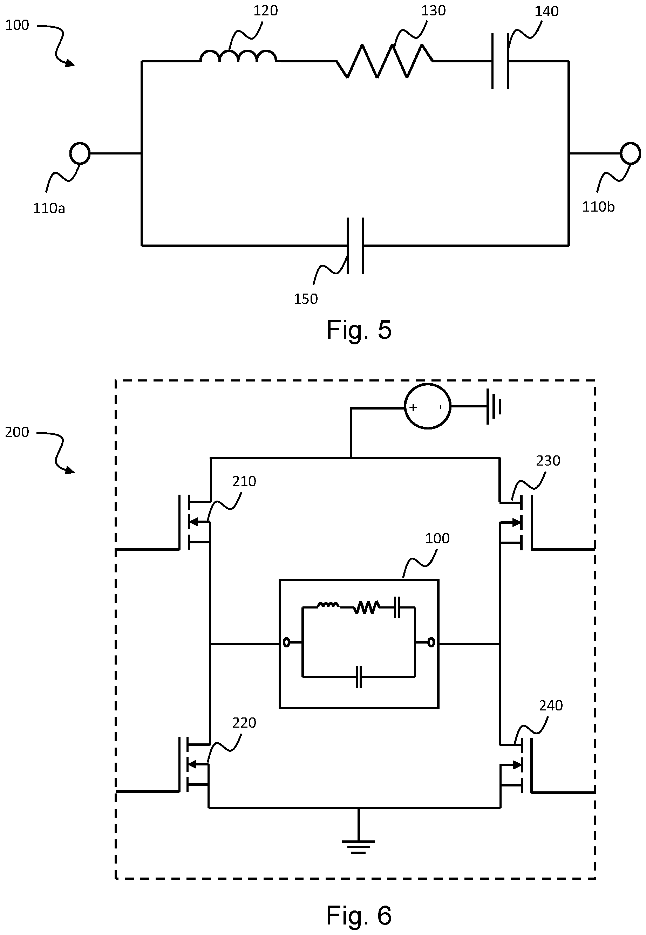

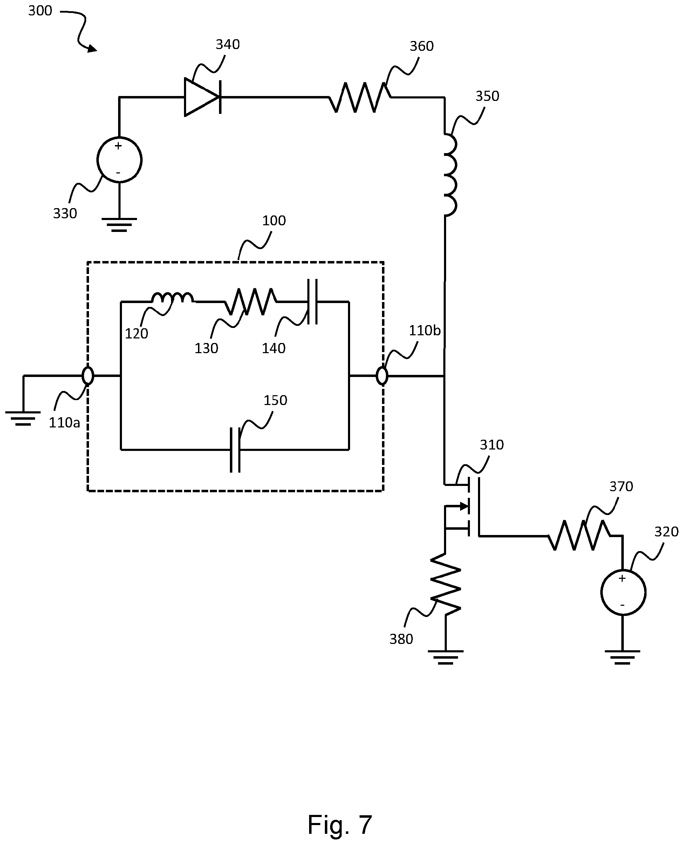

Fig. 5 shows an example of a circuit for modelling the behaviour of a piezoelectric transducer 100 at the resonant frequency of the piezoelectric transducer 100. Example circuits for providing a driving signal to the piezoelectric transducer 100 are described below with reference to Figures 6 and 7.

The circuit includes a set of components connected in series with each other between a pair of terminals 110a, 110b, including: an inductor 120; a resistor 130; and an in-series capacitor 140. The set of series components are connected in parallel with an in-parallel capacitor 150. Each of the components of the circuit model different aspects of the electrical and mechanical behaviour of the piezoelectric transducer 100.

The mechanical vibration of the piezoelectric transducer 100 is modelled by the inductive reactance of the inductor 120, when the frequency of the electric signal driving the piezoelectric transducer 100 is at, or near, the resonant frequency of the piezoelectric transducer 100.

Internal losses associated with the operation of the piezoelectric transducer 100, such as mechanical damping and dielectric losses within the piezoelectric crystal of the transducer 100 are modelled by the resistor 130. The resistance value of the resistor 130 is linked to the quality factor (or Q-factor) of the resonance of the piezoelectric transducer 100, which affects the amplitude and the sharpness of the resonance peak in the transducer's 100 frequency response.

Capacitive mechanical and electrical characteristics of the piezoelectric transducer 100 are modelled by the in-series capacitor 140.

Inherent dielectric properties of the material forming the piezoelectric transducer, e.g., due to the structure of the piezoelectric material between electrodes of the transducer 100 are modelled by the in-parallel capacitor 150. This inherent "parallel" capacitance significantly influences the resonance behaviour of the piezoelectric transducer 100, for example, by affecting the total impedance of the circuit at resonance when combined with the inductive and resistive elements (as modelled by the inductor 120 and the resistor 130).

As an example, the circuit of Figure 5 may be suitable for modelling a typical piezoelectric transducer 100 with a resonance frequency of approximately 3 MHz, by providing the inductor 120 with an inductance of 3 µH, the resistor 130 with a resistance of 4 Ω, the in-series capacitor 140 with a capacitance of 938 pF, and the in-parallel capacitor 150 with a capacitance of 1 nF.

Fig. 6 shows a portion of a conventional driving circuit 200 for driving a piezoelectric transducer 100 using an H-bridge. The H-bridge is defined by four switches 210, 220, 230, 240 arranged in an 'H-shaped' arrangement around the piezoelectric transducer 100. In the example shown in Fig. 6, the four switches 210, 220, 230, 240 are each defined by a respective MOSFET. The H-bridge of Fig. 6 is useful for rapidly changing the polarity of a voltage applied to the piezoelectric transducer 100, thereby driving piezoelectric vibrations in the transducer 100.

H-bridge circuits such as the one depicted in Fig. 6 may face challenges in the context of a user device such as the aerosol-generating apparatus 1 described herein.

For example, high frequency switching of the four (MOSFET) switches 210, 220, 230, 240 may result in significant power and heat dissipation, generating considerable amounts of heat. This heat can degrade component performance over time and shorten the lifespan of the whole H-bridge, including the piezoelectric transducer 100. Moreover, the power dissipation may represent an undesirable inefficiency in the circuit performance of the H-bridge.

There may also be a risk of a latch-up type short-circuit in which one or more parts of the H-bridge circuit become uncontrollably conductive, thereby compromising the circuit's reliability. In the extreme, latch-up can lead to total circuit failure.

Electromagnetic interference may also be a concern when considering the implementation of a H-bridge. The rapid switching inherent in the operation of the four (MOSFET) switches 210, 220, 230, 240 can generate interference that can disrupt the operation of other electronic components/devices in the vicinity of the H-bridge.

Additionally, the operation of the piezoelectric transducer 100 (or indeed any component having an inductive load), can lead to high-voltage spikes in the current flowing through the circuit. Such spikes risk causing severe damage to the transistors used to embody the four MOSFET switches 210, 220, 230, 240 of the H-bridge shown in Fig. 6.

Furthermore, to induce high-frequency (e.g., ultrasonic) vibrations in the piezoelectric transducer 100, very precise and potentially complex timing control of the H-bridge is required. In particular, if both the first and second switches 210, 220, both the first and third switches 210, 230, both the second and fourth switches 220, 240 or both the third and fourth switches 230, 240 are open at the same time, there is a significant risk of shoot-through, or crossover, current that risks damaging the switches as the shoot-through current passes through and reduces the power efficiency of the H-bridge.

Fig. 7 shows an example of an improved driving circuit 300 for driving a piezoelectric transducer 100 using a single (MOSFET) switch 310.

The driving circuit 300 of Fig. 7 comprises a gate power source 320 configured to controllably apply a voltage to the gate of the MOSFET switch 310 to controllably open and close the MOSFET switch 310. The gate power source 320 may be connected to a clock, or may be an oscillator circuit so as to cyclically open and close the MOSFET switch 310 at a selected frequency.

The driving circuit 300 further comprises a driving power source 330 configured to supply power through the driving circuit 300. When the MOSFET switch 310 is closed, the current supplied by the driving power source 300 bypasses the piezoelectric transducer and flows into the source of the MOSFET switch 310 and out from the drain of the MOSFET switch 310 to ground. The driving power source 330 may be the power supply 2 discussed above in relation to Fig. 1.

When the MOSFET switch 310 is open, the current supplied by the driving power source 300 flows through the piezoelectric transducer 100 to ground, thereby inducing vibration in the piezoelectric transducer.

Application of a current to a piezoelectric transducer 100 induces a mechanical response in the transducer 100. Typically, the current applied to the piezoelectric transducer 100 is an alternating current so as to induce oscillatory vibrations in the piezoelectric transducer 100. Upon application of a current in a first polarity, opposite faces of the piezoelectric crystal of the transducer 100 respond by expanding, or bulging, outwards to define respective convex surfaces. Conversely, upon application of current in a second polarity opposite to the first polarity, the opposite faces of the piezoelectric crystal of the transducer 100 respond by contracting, or drawing, inwards to define respective concave surfaces. In the context of an aerosol-generating apparatus 1, it may be advantageous to only drive the piezoelectric transducer 100 in the first polarity so that physical contact between the transducer 100 and the at least some of the liquid precursor 6 can be maintained. Maintaining this physical contact improves the power efficiency of the inducement of cavitation in the liquid precursor 6, and therefore improves the efficiency of the generation of the aerosol. To this end, the driving signal provided by the driving power source 330 is preferably a direct current power source oscillating, at the piezoelectric transducer 100, between a maximum amplitude and a minimum (zero) amplitude with a frequency corresponding to the switching frequency of the MOSFET switch 310.

The driving circuit 300 may further comprise an inductor 350 connected in series with the piezoelectric transducer. The inductor 350 is arranged and configured with an inductance suitable for smoothing the current profile of the signal provided by the driving power source 330 such that the piezoelectric transducer 100 is not subjected to abrupt step-changes in the voltage and current flowing therethrough. This smoothing of the current profile consequently reduces the risk of damage to the piezoelectric transducer by reducing the risk of harmful voltage spikes.

The driving circuit 300 may further comprise one or more resistors 360, 370, 380 configured to limit the current flowing through the driving circuit.

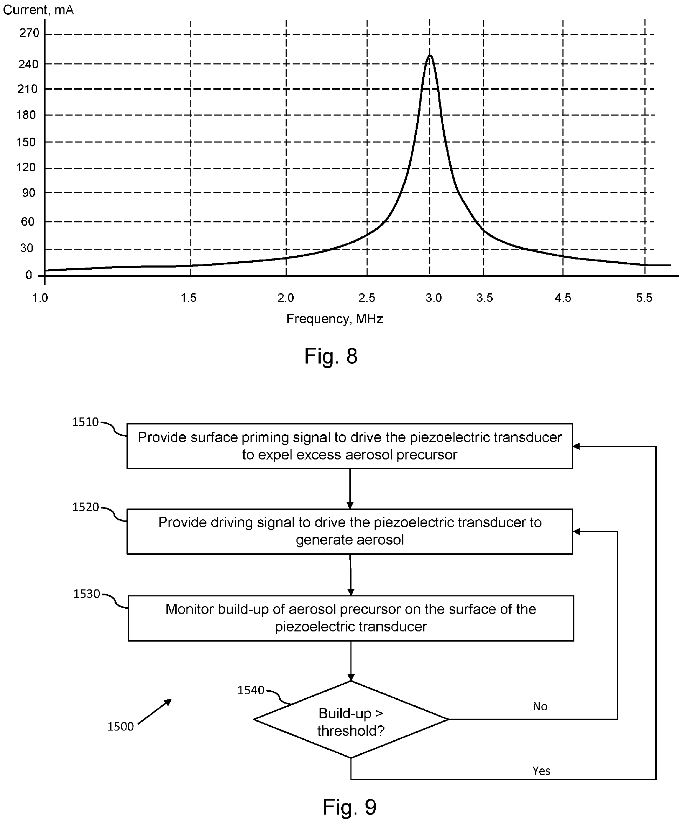

Fig. 8 shows an exemplary frequency response in the current flowing through a piezoelectric transducer 100. As can be seen in the current response of the piezoelectric transducer 100, depicted in Fig. 8, this particular piezoelectric transducer 100 exhibits a resonant response when a driving signal having a driving frequency of approximately 3 MHz. In other words, the fundamental resonant frequency of piezoelectric transducer 100 is approximately 3 MHz.

Fig. 9 shows a method 1500 of controlling an aerosol generating apparatus, such as the aerosol generating apparatuses 1 described herein.

The method 1500 comprises, in an operation 1510, providing a surface priming signal to drive the piezoelectric transducer 100 to expel excess aerosol precursor from the surface of the piezoelectric transducer 100. In some examples, driving the piezoelectric transducer according to the surface priming signal may further expel excess aerosol precursor from at least a portion of a wick of the aerosol generating apparatus 1. The wick of the aerosol generating apparatus 1 is arranged to convey aerosol precursor 6 from the tank 32 to the piezoelectric transducer 100 for aerosolization according to the methods described herein and in Kooij *et al.,* the entirety of which is incorporated by reference.

The method 1500 further comprises, in an operation 1520, providing a driving signal to drive the piezoelectric transducer 100 so as to generate aerosol from received aerosol precursor received on the surface of the piezoelectric transducer 100.

The method 1500 further comprises, in an operation 1530, monitoring a build-up of aerosol precursor 6 on the surface of the piezoelectric transducer 100. Monitoring the build-up of aerosol precursor 6 on the surface of the piezoelectric transducer 100 may involve monitoring a change in one or more of: a mass of received aerosol precursor 6 on the surface of the piezoelectric transducer 100, a volume of received aerosol precursor 6 on the surface of the piezoelectric transducer 100, a volume of aerosol generated per puff of the aerosol generating apparatus 1, when in use, and/or a concentration of aerosol generated per puff of the aerosol generating apparatus 1, when in use.

The method 1500 further comprises, in an operation 1540, determining whether the build-up of aerosol precursor 6 on the surface of the piezoelectric transducer 100 has crossed (e.g. is greater than) a predetermined threshold.

Crossing the predetermined threshold may involve a mass of received aerosol precursor 6 on the surface of the piezoelectric transducer 100 going above a predetermined mass threshold.

Additionally or alternatively, crossing the predetermined threshold may involve a volume of received aerosol precursor 6 on the surface of the piezoelectric transducer 100 going above a predetermined volume threshold.

Additionally or alternatively, crossing the predetermined threshold may involve a volume of aerosol generated per puff of the aerosol generating apparatus 1 going below a predetermined aerosol volume threshold.

Additionally or alternatively, crossing the predetermined threshold may involve a concentration of aerosol generated per puff of the aerosol generating apparatus going below a predetermined aerosol concentration threshold.

If the threshold(s) is not crossed, then the method may involve repeating the providing of the driving signal and the monitoring of the build-up of aerosol precursor 6 (as in operations 1520 and 1530).

If the threshold(s) is crossed, then the method may involve providing the surface priming signal (as in operation 1510) to expel excess aerosol precursor from the surface of the piezoelectric transducer 100 and, optionally, a portion of the wick.

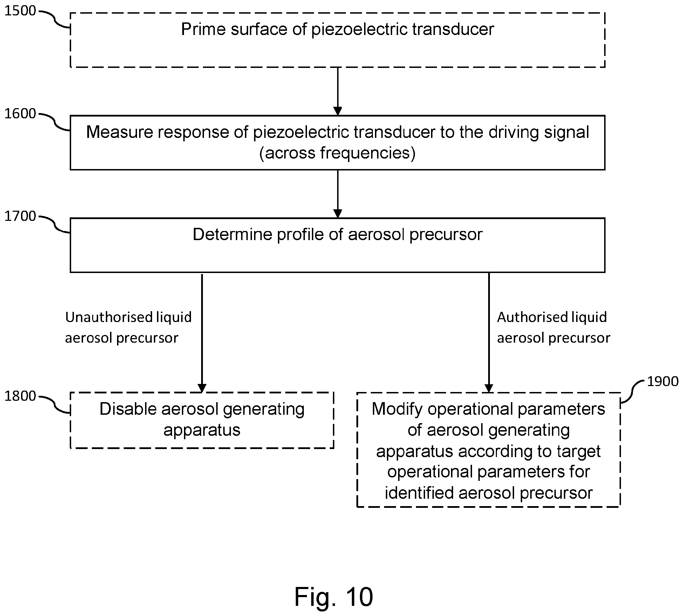

Fig. 10 shows another method of controlling an aerosol generating apparatus 1.

Fig. 10 may comprise, in an operation 1500, carrying out the method of Fig. 9 to prime the surface of the piezoelectric transducer 100 so that the measurements and determinations described below are properly calibrated.

Fig. 10 comprises, in an operation 1600, measuring a response of the piezoelectric transducer 100 to one or more driving signals across a plurality of driving frequencies, for example by measuring the piezoelectric transducer's 100 response as the driving signal is scanned across a range of frequencies.

Measuring the response of the piezoelectric transducer 100 may involve measuring one or more of the piezoelectric transducer's current response, impedance response or voltage response across the plurality or range of frequencies.

Fig. 10 further comprises, in an operation 1700, determining a profile of the aerosol precursor 6.

Determining a profile of the aerosol precursor 6 may involve comparing the measured response of the piezoelectric transducer 100 with one or more reference responses that may, for example, be stored in a database of reference responses - each of the reference responses being associated with an authorised aerosol precursor that is authorised for use with the aerosol generating apparatus 1.

Fig. 10 may further comprise, in an operation 1800, if it is determined that the aerosol precursor 6 is not an authorised aerosol precursor (i.e., is an unauthorised aerosol precursor such as an illicit or counterfeit aerosol precursor) disabling the aerosol generating apparatus 1. This disabling may be carried out to prevent damage to the aerosol generating apparatus and/or to the health of a user of the aerosol generating apparatus.

Fig. 10 may further comprise, in an operation 1900, if is determined that the aerosol precursor 6 is an authorised aerosol precursor, modifying one or more operational parameters of the aerosol generating apparatus according to target operational parameters for the identified authorised aerosol precursor 6.

The one or more target operational parameters may, for example, be stored in the database with the one or more reference responses. The one or more operational parameters may include one or more of a driving frequency, a driving duty cycle, and/or a driving power of the driving signal.

### REFERENCES

The entirety of the following documents, which are referenced in the present disclosure, are incorporated by reference into the present disclosure:
- Kooij, S., Astefanei, A., Corthals, G.L. et al. Size distributions of droplets produced by ultrasonic nebulizers. Sci Rep 9, 6128 (2019). https://doi.org/10.1038/s41598-019-42599-8

## Claims

1. A method of controlling an aerosol generating apparatus (1) to modify the delivery of aerosol generated by the aerosol generating apparatus, wherein the aerosol generating apparatus includes: a tank (32) for containing an aerosol precursor (6), a piezoelectric transducer (100) arranged to receive aerosol precursor from the tank and configured to induce cavitation in the received aerosol precursor to thereby generate an aerosol from the received aerosol precursor, wherein the method comprises:
providing, to the piezoelectric transducer (100), a surface priming signal to drive the piezoelectric transducer so as to expel excess aerosol precursor from a surface of the piezoelectric transducer.

2. The method according to any preceding claim, wherein driving the piezoelectric transducer (100) to expel excess aerosol precursor from the surface of the piezoelectric transducer further expels excess aerosol precursor from at least a portion of a wick of the aerosol generating apparatus (1), wherein the wick is arranged to convey the aerosol precursor (6) from the tank (32) to the piezoelectric transducer.

3. The method according to claim 1 or 2, the method further comprising: providing, to the piezoelectric transducer (100), a driving signal to drive the piezoelectric transducer so as to generate an aerosol from the received aerosol precursor.

4. The method according to claim 3, wherein a power level of the surface priming signal is greater than a power level of the driving signal.

5. The method according to claim 3 or 4, the method further comprising: monitoring, while the aerosol generating apparatus is in use, a build-up of aerosol precursor on the surface of the piezoelectric transducer (100), and providing the surface priming signal to drive the piezoelectric transducer in response to the aerosol precursor crossing a predetermined threshold.

6. The method according to claim 5, wherein the predetermined threshold is one or more of:
a predetermined volume of aerosol precursor on the surface of the piezoelectric transducer (100);
a predetermined mass of aerosol precursor on the surface of the piezoelectric transducer;
a volume of aerosol generated per puff of the aerosol generating apparatus, when in use; and/or
a concentration of aerosol generated per puff of the aerosol generating apparatus, when in use.

7. The method according to any of claims 3 to 6, the method further comprising:
measuring a response of the piezoelectric transducer (100) to the driving signal; and
determining, based on the measured response, a profile of the aerosol precursor (6).

8. The method according to claim 7, wherein determining the profile of the aerosol precursor involves comparing the measured response of the piezoelectric transducer (100) with one or more reference responses,
wherein each of the one or more reference responses corresponds to a respective authorised aerosol precursor that is authorised for use with the aerosol generating apparatus (1), and
wherein determining the profile of the aerosol precursor (6) further involves verifying, based on the comparing the measured response with the one or more reference responses, whether the aerosol precursor is an authorised aerosol precursor.

9. The method according to claim 8, the method further comprising: if it is determined that the aerosol precursor (6) is not an authorised aerosol precursor, disabling the aerosol generating apparatus (1).

10. The method according to claim 8 or 9, the method further comprising: if it is determined that the aerosol precursor (6) is one of the one or more authorised aerosol precursors, modifying one or more operational parameters of the aerosol generating apparatus (1) according to stored target operational parameters associated with the one of the one or more authorised aerosol precursors.

11. The method according to any of claims 7 to 10, wherein measuring the response of the piezoelectric transducer (100) involves measuring one or more of:
the current response of the piezoelectric transducer;
the impedance response of the piezoelectric transducer; and/or
the voltage response of the piezoelectric transducer
to one or more driving signals, each of the one or more driving signals having a different driving frequency.

12. The method according to any of claims 7 to 10, wherein measuring the response of the piezoelectric transducer (100) involves scanning the driving signal across a range of frequencies, and measuring one or more of:
the current response of the piezoelectric transducer;
the impedance response of the piezoelectric transducer; and/or
the voltage response of the piezoelectric transducer
across the range of frequencies.

13. An aerosol generating system (1) comprising:
a tank (32) for containing an aerosol precursor (6);
a piezoelectric transducer (100) arranged to receive liquid aerosol precursor (6) from the tank (32) on a surface of the piezoelectric transducer, wherein the piezoelectric transducer is configured to induce cavitation in the received liquid aerosol precursor to thereby generate an aerosol from the received liquid aerosol precursor; and
electrical circuitry configured to carry out the method of any preceding claim.

14. A computer-readable medium comprising instructions that, when carried out by a computer, cause the computer to carry out the method of any of claims 1 to 12.

15. Electrical circuitry for an aerosol generating system, the electrical circuitry being arranged to perform the method of any of claims 1 to 12.
